# EUROPEAN PATENT SPECIFICATION

(11) **EP 2 289 587 B1**
(45) Date of publication and mention of the grant of the patent: **06.01.2016**
(21) Application number: 10185014.7
(22) Date of filing: 16.06.2000
(51) Int. Cl.: A61M 16/06

(54) **Mask and headgear with connector**
Maske und Kopfgurt mit Anschlussteil
Masque et casque avec connecteur

(30) Priority: 18.06.1999 AU PQ104099; 18.06.1999 AU PQ191699; 16.12.1999 US 115618; 15.02.2000 US 504220
(43) Date of publication of application: 02.03.2011
(62) Divisional of application: 05026584.2
(73) Proprietor: ResMed Limited, Bella Vista, NSW 2153 (AU)
(72) Inventor: Gunaratnam, Michael Kassipillai, Marsfield NSW 2122 (AU); Kwok, Philip Rodney, Chatswood NSW 2113 (AU)
(74) Representative: Vossius & Partner Patentanwälte Rechtsanwälte mbB

(56) References cited:
- WO-A-98/48878
- FR-A- 2 727 836
- GB-A- 684 788
- GB-A- 704 167
- GB-A- 2 293 410
- US-A- 2 097 676
- US-A- 2 292 568

## Description

### Field of the Invention

The invention relates to a connector for use with a mask and headgear suitable for the delivery of breathable gases to a patient for the treatment of sleep disordered breathing (SDB).

### Background of the Invention

Respiratory masks used in the treatment of SDB may comprise either a nasal mask, designed to fit over a patient's nose, or a full face mask designed to fit over the nose and mouth of the patient. Air or other breathable gas is supplied by a blower and passed along a flexible conduit to the mask.

The mask generally comprises a relatively rigid shell, termed a frame, which defines a rearwardly opening cavity covering the patient's nose and/or mouth and a soft portion, termed a cushion, which spaces the frame away from the face for comfortable contact.

The masks are typically held in place using headgear, the mask and headgear being joined using some form of connector.

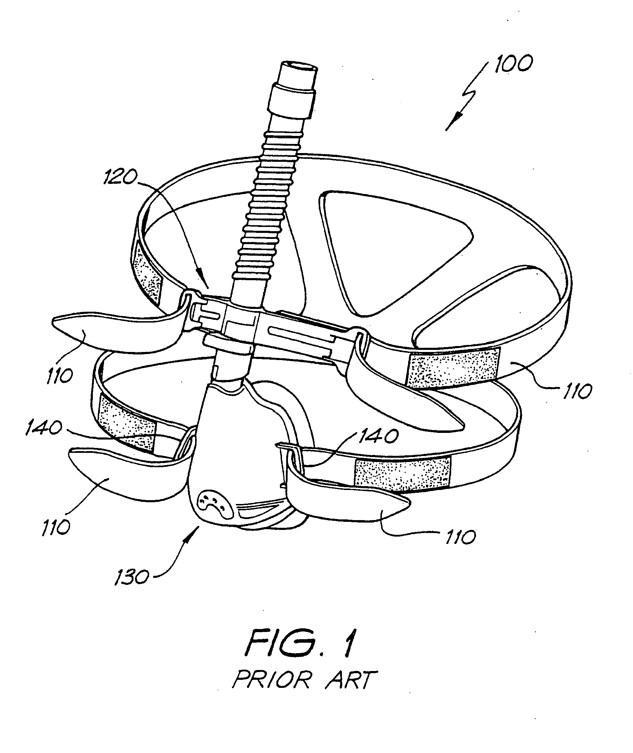

One known example is the Mirage® Mask (ResMed Limited), shown in Fig. 1. In this case, the headgear 100 is constructed from fabric and includes a rear portion, which engages the region near the occiput of the patient, and four straps 110 which are secured to a forehead support 120 (2 straps) and nasal mask frame 130 (2 straps). The straps include hook and loop material, such as Velcro(TM) on one side. The mask frame and forehead supports include loops 140 through which a strap can pass. In order to secure the mask in place on a patient's head, the four straps are passed through the four corresponding loops and held in place at an appropriate length by the hook and loop material. The patient can adjust the length of the material in order to secure a good fit of the mask. In some cases, it can require considerable adjustment to find the optimal length of straps which is required. A difficulty with this connector is that if the patient removes the mask by loosening the straps, they will lose what may have been a good fit of the mask the next time it is being used. In this case they will need to repeat the adjustment step.

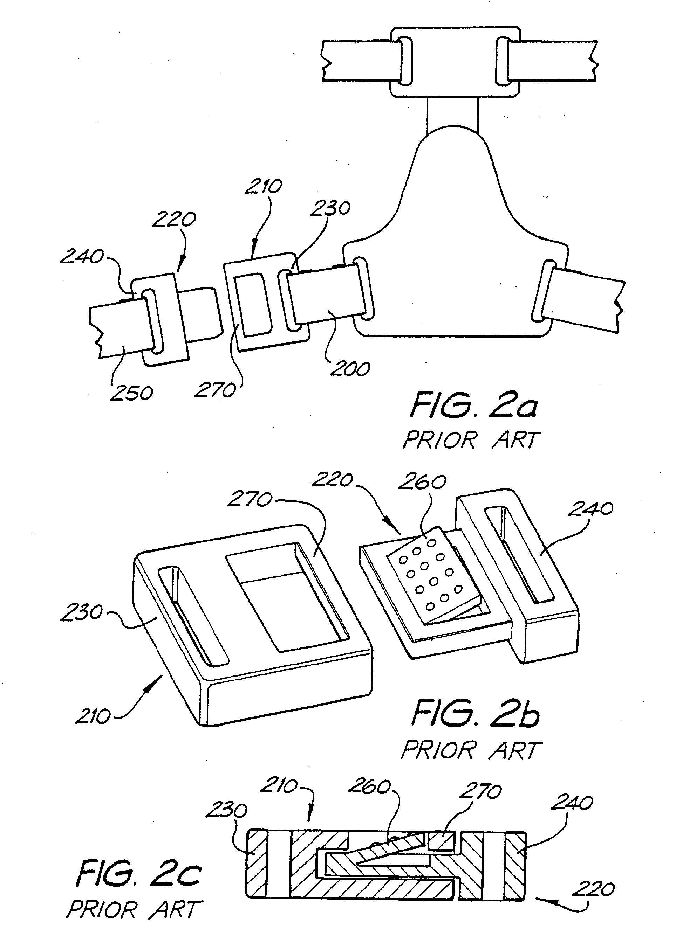

Another known manner of connecting the mask and headgear is shown in Figs. 2a, 2b and 2c. In this headgear, a short strap length 200 is secured to one side of the mask, with a two-part, press-xelease connector 210, 220 attaching this to the strap 250 of the headgear. One disadvantage of the approach of using this arrangement is that the connector may be difficult to release because the connector is free to move relative to the mask frame. A further disadvantage of this arrangement is that the connector may be in contact with the patient's face which may lead to discomfort in use, particularly if they sleep on their side.

More detailed views of this prior art connector are shown in Figs. 2b and 2c. Each part of the connector includes a bar 230, 240 behind which a respective one of the straps 200, 250 may be passed. The male portion 220 of the connector includes a resilient cantilever 260 which is captured behind a bar 270 on the female portion 210. The cantilever is depressed to engage and disengage the connector. A further disadvantage of this particular connector, best seen in Fig. 2b, is that it may be awkward to disengage the connector because of the close positioning of the bar 270 to the cantilever 260.

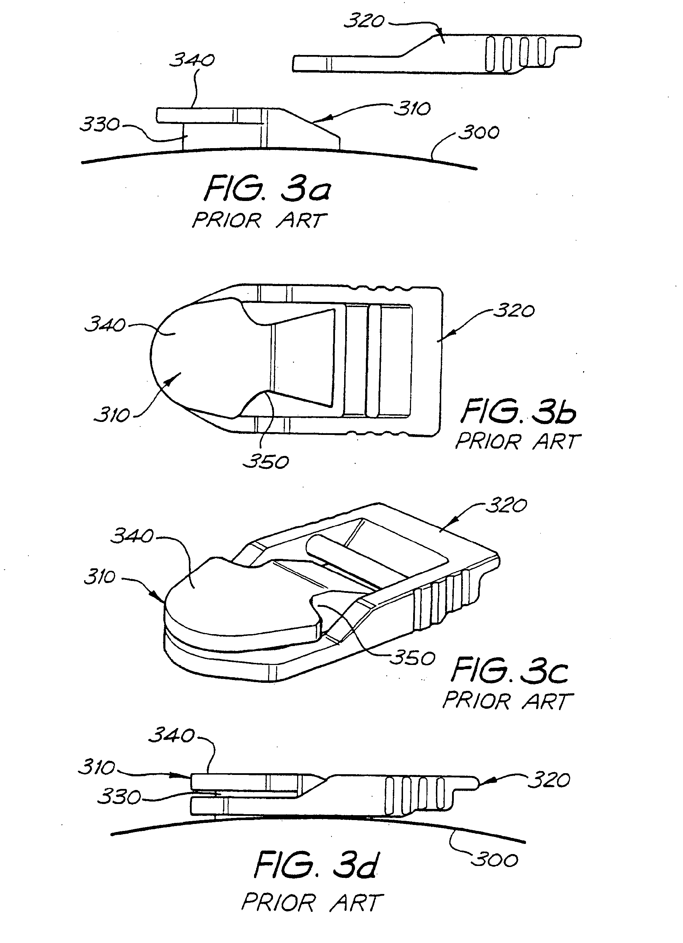

Another known mask and headgear connector is shown in Fig. 3a to Fig. 3d. This comprises a flexible part 310 positioned on the outer surface of a flexible mask frame 300 and a rigid part 320 formed generally as a D-ring with a loop to which the headgear strap is attached. The flexible part consists of a base 330 supporting an upper portion 340 (best illustrated in Fig. 3a) which overhangs the base portion 330 and has a narrow central region 350. In order to engage and disengage the connector, the flexible part 310 must be deformed whilst the D-ring of the rigid part is pushed over the upper portion 340 to engage below the overhanging ledge. This arrangement is awkward to engage and disengage and typically requires two hands.

Another known mask and headgear connector consists of hooks on the end of the headgear straps and corresponding holes in the mask frame. To engage the mask and headgear connector, the hook is passed through one of the corresponding holes on the headgear. This arrangement is also awkward to engage and disengage and typically requires two bands. Also, it is possible for the hooks to disengage during sleep as there is no locking means for the connection.

There is a need for a connector arrangement which is simple and quick to operate.

### Summary of the Invention

The present Invention Is defined by the subject-matter of the Independent claim. The invention provides, in one preferred form, a respiratory mask and headgear combination comprising a respiratory mask having a rigid mask frame, adjustable headgear for securing said mask on a patient, said headgear including at least one attachment strap, said mask frame having rigidly secured thereto a rigid first connector, further comprising a second connector adapted for releasable mating with said first connector, said second connector having means for connection of said attachment strap of the headgear.

There is further discussed a respiratory mask and headgear combination adapted for single-banded disengagement, comprising a rigid mask frame, adjustable headgear for securing said mask on a patient, said headgear including at least one attachment strap, said mask frame having rigidly secured thereto a rigid first (female) connector, further comprising a second (male) connector connected to said strap of said headgear, said second connector being adapted for releasable mating with the first connector and having first and second gripping surfaces positioned for gripping of said second connector between a thumb and finger of a patient's hand and release means positioned for operation by another finger of the patient's hand.

### Brief Description of the Drawings

Further embodiments of the invention will now be described with reference to the accompanying drawings, in which:
Fig. 1 shows the prior art Mirage® mask and headgear assembly.
Fig. 2a shows a front view of a prior art mask and headgear assembly.
Fig. 2b shows further detail of the prior art mask and headgear connector shown in Fig. 2a.
Fig. 2c shows a cross-sectional view of the prior art mask and headgear connector shown in Fig. 2b.
Fig. 3a shows a side view of another prior art mask and headgear connector where the two parts are not engaged.
Fig. 3b shows a top view of the prior art connector shown in Fig. 3a, where the two parts are engaged.
Fig. 3c shows a perspective view of the prior art connector shown in Fig. 3a, where the two parts are engaged.
Fig. 3d shows a side view of the prior art connector shown in Fig. 3a, where the two parts are engaged.
Fig. 4 shows a front view of a mask frame incorporating a female portion of the connector according to one embodiment of the invention.
Fig. 5 shows a perspective view of the mask frame of Fig. 4.
Fig. 6a shows a front view of a male portion of the connector adapted for use with the mask frame of Figs. 4 and 5.
Fig. 6b shows a side view of Fig. 6a.
Fig. 6c shows a sectional view along A-A of Fig. 6a.
Fig. 6d shows a perspective view of Fig. 6d.
Fig. 7a and 7b show an embodiment of the invention where the cross-bar on the male portions of the connectors have different angles.

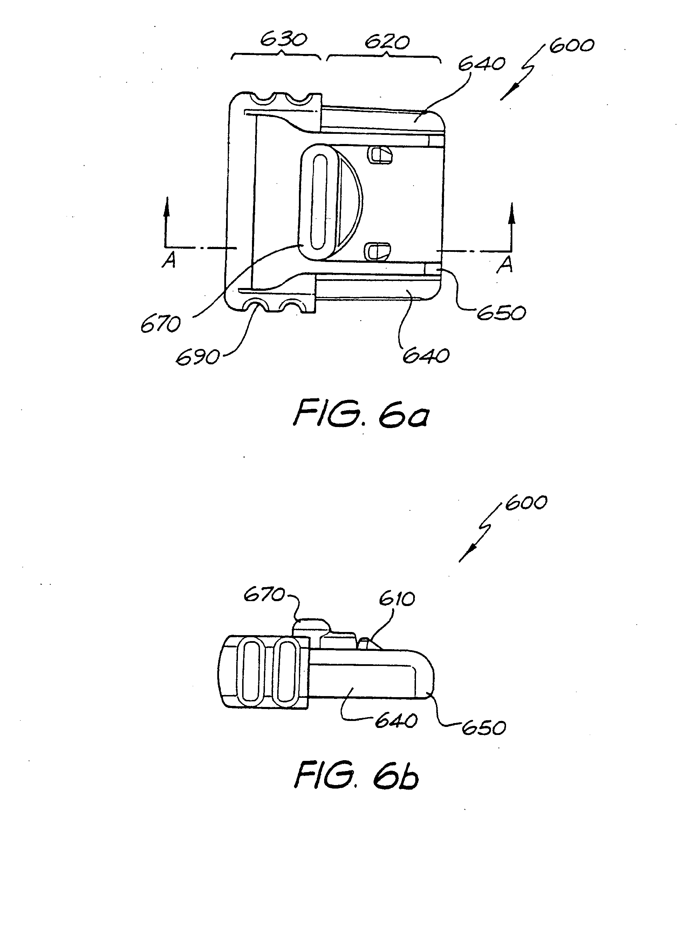

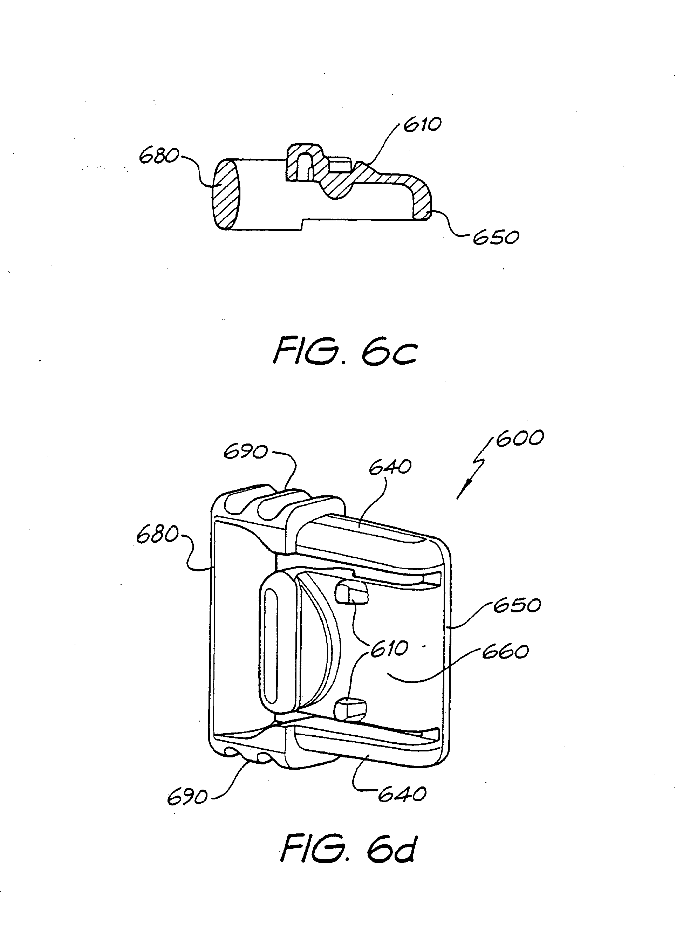

In Figs. 6a to 6d, dimensions shown are in millimeters.

### Detailed Description of the Invention

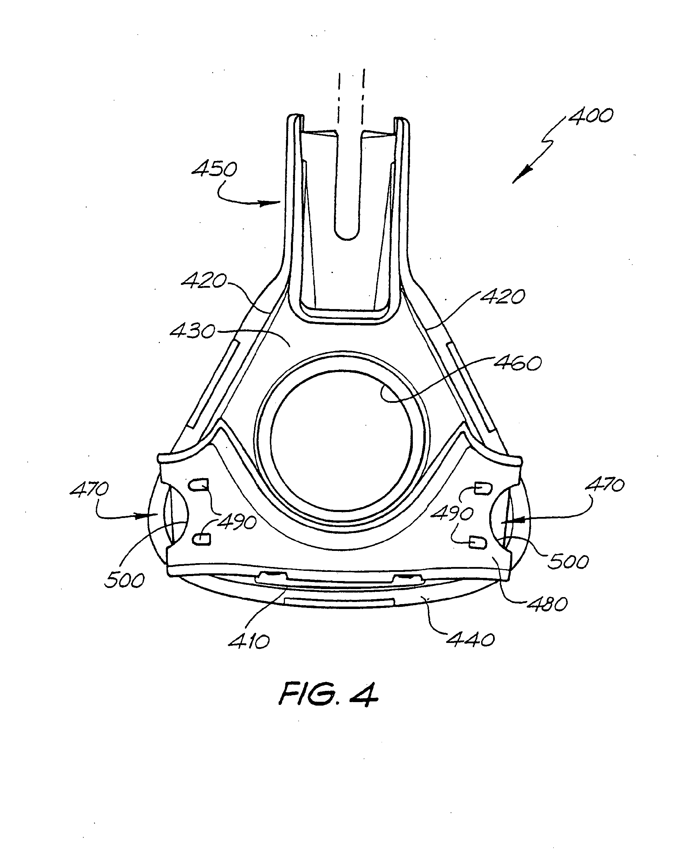

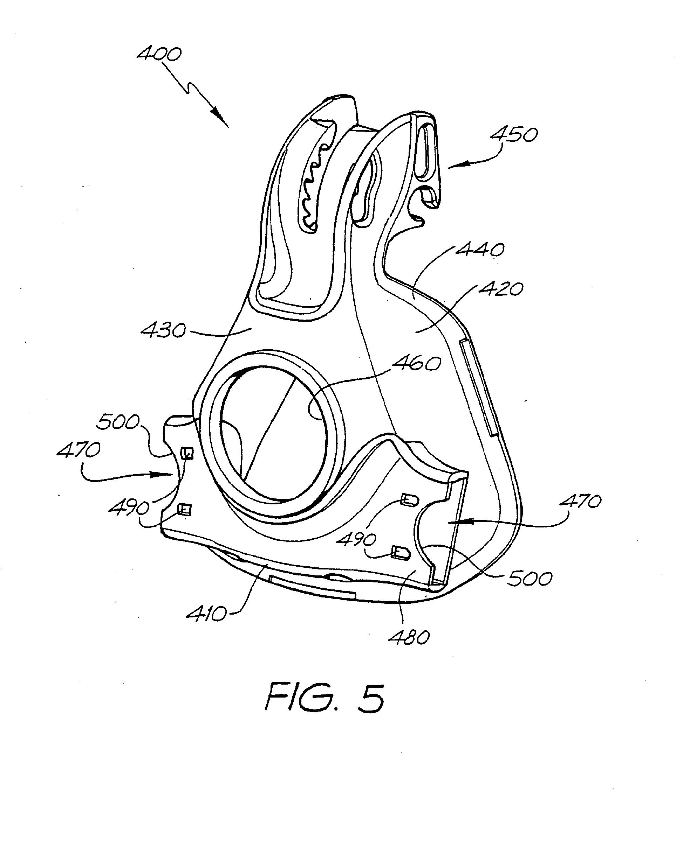

Figs. 4 and 5 illustrate a mask frame 400 for a nasal mask, formed as a moulded shell of polycarbonate or similar rigid material, which acts as a body onto which the other components of the mask are attached.

The frame 400 is generally triangular in front view, having a base 410, a pair of inclined side walls 420 extending towards an apex and a front wall 430. The frame defines a mask cavity covering the patient's nose, and is open at its rear. A rim 440 at the rear edge of the base 410 and side walls 420 approximates the contours of the patient's face and is adapted for attachment of a soft mask cushion (not shown) to space the frame away from the patient's face for sealing and comfort. The apex of the frame has an extension 450 for attachment of a forehead support (not shown).

In the illustrated mask frame, a gas inlet aperture 460 is formed in the front wall 430, for connection of a gas supply conduit or similar, which may include an elbow connector (not shown) pivotably connected to the frame. In other forms of mask, the gas inlet aperture may be formed at the apex of the frame.

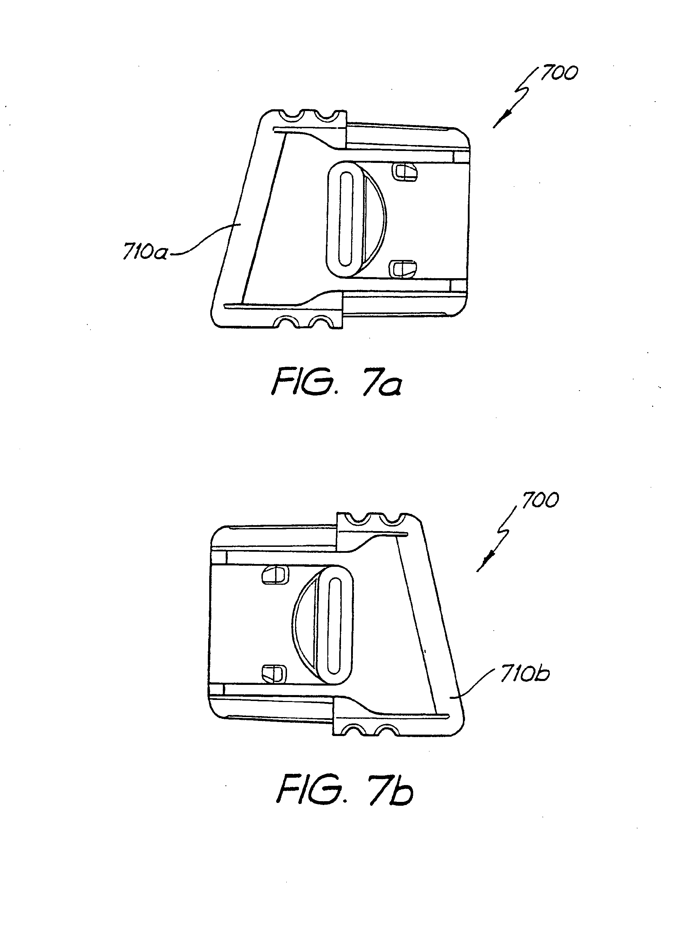

Integrally moulded into the lower corner regions of the rigid mask frame are a pair of female connector parts 470, for receiving the leading portions of the male connector parts 600, 700 illustrated in Figs. 6a to 6d, or Figs. 7a and 7b. These female connector parts open generally to the side of the mask, but are angled slightly downwards and rearwards (relative to the orientation of the mask frame) so that they hold the male connectors approximately parallel to that part of the patient's cheek region which the connectors overlie. The connectors are held clear of the patient's face by the rigid mask frame 400.

The female connectors 470 each define a recess which opens towards a side of the mask and which approximates a rounded-cornered rectangular in end view, adapted to provide a close fit with the corresponding male connectors 600,700 when engaged. These end shapes are preferably slightly asymmetric (keyed) so as to prevent upside-down insertion of the male connector.

A front wall 480 of the female connector has a pair of sockets 490 at least on its inner surface, for receiving respective lugs 610 of the male connector 600. The end of the front wall may also have a curved cut-out portion 500, as will be described below.

A corresponding male connector part 600 is illustrated in Figs. 6a to 6d.

The male connector has a leading portion 620 which is received in the recess of the female connector 470, and a trailing portion 630 which remains outside the recess. The leading portion includes upper and lower side beams 640 connected by a cross-piece 650 at their leading ends, and a resiliently biased cantilever member 660 depending from the cross-piece 650 and extending back towards the trailing portion. The cantilever has on its front surface the lugs 610, and a ridge 670 at its trailing edge.

The locating lugs 610 have a rounded wedge profile so as to allow reduce wear and provide smooth engagement. The wedge profile does allow insertion of the male connector 600 into the female connector 470 on the mask without depression of the cantilever, although in preferred operation the patient will depress the cantilever by pushing on ridge 670 for both engagement and disengagement. Once the male connector has been inserted sufficiently to snap the locating lugs 610 into engagement with corresponding sockets 490 of the female connector, disengagement can occur only by depression of the cantilever.

The trailing portion 630 of the male connector has a cross-bar 680 forming a loop through which the headgear strap can be passed and adjusted for proper fit. Upper and lower sides 690 of the trailing portion have grooves or other formations to make it more easily gripped by the patient's fingers, while the distance between the leading side of the cross-bar 680 and the trailing edge and ridge 670 of the cantilever is preferably at least 5mm to allow easy connection of the strap and provide sufficient space for a finger to push on the cantilever ridge, even when the strap is attached. The arcuate cut-out 500 in the female connector front wall allows clearance for an end of the finger to overhang the ridge 670 when the ridge is being contacted by the pad of the finger.

Figs 7a and 7b illustrate a male connector 700 which is a variation on that of Figs 6a to 6d, in that the cross bars 710a, 710b for attachment of the headgear strap are set at an angle.

The shape, dimensions and position of the connectors may be optimised for most convenient use. For example, the male connector may be approximately 20 mm wide, 25 mm long and 10 mm thick. The cantilever 660 is approximately 15 mm long. The locating lugs 610 are approximately 3 mm long 2 mm wide and 1 mm high, and the ridge is approximately 14 mm wide and has a length of approximately 5 mm. Other dimensions of the male portion are indicated in Fig. 6a to 6c.

The female connectors 470 are oriented on the mask frame so that in use the straps of the headgear are aligned to be approximately parallel with the sides of the face with which they make contact. When viewed from the front of the mask (the view in Fig. 4), the tops of the left and right hand side male connector's cross-bars 680 are further apart than the bottoms of the cross-bars. When viewed from the top the mask, the connectors are aligned to be positioned on an arc of an ellipse. An advantage from this arrangement is that when straps are connected and tensioned, the line of force will be more evenly transferred from the mask to the frame.

Both portions of the connector are preferably constructed from polycarbonate, such as Makrolon 2458 polycarbonate from Bayer, or similar rigid plastics material. The female portions of the connectors are clear and desirably are integrally moulded with the mask frame which is formed of the same polycarbonate material, thus ensuring a permanent, rigid attachment of the female connector to the mask frame and minimising the number of separate parts. The male portions may be frosted or textured. An advantage from using a frosted or slightly textured surface is that the male portion may be easier to distinguish from the female portion by touch. This is an advantage in the dark, the typical time when the mask is being used by a patient.

The configuration thus allows single-hand operation of the connector using, for example, thumb and index finger to grip the grooved sides 690 of the male connector and the middle finger to depress the cantilever. The connectors may be located on both the left and right hand side of the mask. In this way persons may use which ever side is most convenient to them. Alternatively the frame may be farmed with only one connector, to reduce manufacturing costs.

In one unillustrated embodiment, the press release mechanism may be adapted to be operated by pressing the top and/or bottom sides of the male connector.

By rigidly forming one part of the connector onto the mask frame, the patient does not need to use both hands to disconnect the mask for removal. Thus, the arrangement facilitates quick and easy disconnection even though the connector will be out of patient's line of vision and the patient may be less than fully awake or in an anxious state.

A further advantage of using female portions 470 integrated with the mask frame is that they result in a reduced dead volume of the mask frame.

While particular embodiments of this invention have been described, it will be evident to those skilled in the art that the present invention may be embodied in other specific forms without departing from the essential characteristics thereof. The present embodiments and examples are therefore to be considered in all respects as illustrative and not restrictive, the scope of the invention being indicated by the appended claims rather than the foregoing description, and all changes which come within the meaning and range of equivalency of the claims are therefore intended to be embraced therein.

## Claims

1. A respiratory mask and headgear combination, comprising:
a respiratory mask having a rigid mask frame (400), and
a headgear for securing said mask on a patient, said headgear including at least one attachment strap, said mask frame (400) having rigidly secured thereto a rigid female connector portion (470), further comprising a corresponding male connector portion (600, 700) adapted for releasable mating with said female connector portion (470), said male connector portion (600, 700) being connected to said attachment strap, wherein said female and male connector portions (470, 600, 700) form a press-release connection between said mask frame (400) and said strap, wherein said female connector portion (470) opens generally to the side of the respiratory mask but is angled slightly downwards and rearwards relative to the orientation of the mask frame (400) so that the female connector portion (470) holds said male connector portion (600, 700) approximately parallel to that part of the patient's cheek region which said connector portion (600, 700) overlies.

2. A respiratory mask and headgear combination according to claim 1, wherein the mask frame (400) is generally triangular in front view, having a base (410), a pair of inclined side walls (420) extending towards an apex and a front wall (430).

3. A respiratory mask and headgear combination according to claim 2, wherein the mask frame (400) includes a rim (440) which at the rear edge of the base (410) and side walls (420) approximates the contours of the patient's face and is adapted for attachment of a soft mask cushion to space the frame away from the patient's face.

4. A respiratory mask and headgear combination according to claim 2 or claim 3, wherein the female connector portion (470) is integrally molded into one of the lower corner regions of the mask frame (400).

5. A respiratory mask and headgear combination according to any one of claims 1-4, wherein said male connector portion (600, 700) includes a resiliently biased cantilever member (660) depending from a leading end portion (650) of said male connector portion (600, 700) and having means for locking engagement with female connector portion (470), wherein said cantilever member (660) has a leading end, a trailing end, locking means located intermediate said leading end and trailing end for engaging with said female connector portion (470), and release means located adjacent said trailing end, wherein said release means comprises a raised portion adjacent said trailing end of said cantilever member (660), wherein a space is provided immediately behind said trailing end of the cantilever member (660), wherein said locking means comprises at least one lug (610) on a forward surface of said cantilever member (660), said lug (610) engaging a corresponding socket (490) of said female connector portion (470).

6. A respiratory mask and headgear combination according to claim 5, wherein said at least one lug (610) has a rounded wedge profile.

7. A respiratory mask and headgear combination according to claim 2 or any one of claims 3 to 6 when dependent on claim 2 , wherein the mask frame (440) comprises an extension (450) at its apex configured for attachment of a forehead support.

8. A respiratory mask and headgear combination according to any one of claims 1-7, wherein the female and male connector portions (470, 600, 700) are spaced forwardly of the patient's face by the rigid mask frame (400).

9. A respiratory mask and headgear combination according to any one of claims 1-8, wherein the male connector portion (600, 700) has first and second gripping surfaces positioned for gripping of said male connector portion (600, 700) by a thumb and finger of the patient's hand, and release means positioned for operation by another finger of the patient's hand.

10. A respiratory mask and headgear combination according to claim 5 or any one of claims 6 to 9 when dependent on claim 5, wherein said release means is pressed by said finger to depress the resiliently biased cantilever member (660) of said male connector portion (600, 700).

## Patentansprüche

1. Atemmasken-Kopfband-Kombination, die aufweist:
eine Atemmaske mit einem starren Rahmen (400), und
ein Kopfband zum Anbringen der Maske an einem Patienten, wobei das Kopfband mindestens ein Befestigungsband aufweist, wobei an dem Maskenrahmen (400) ein starrer weiblicher Abschnitt (470) starr angebracht ist, und einen entsprechenden männlichen Abschnitt (600, 700), der zum lösbaren Koppeln mit dem weiblichen Abschnitt (470) geeignet ist, wobei der männliche Abschnitt (600, 700) mit dem Befestigungsband verbunden ist, wobei der weibliche und männliche Abschnitt (470, 600, 700) eine Drucklöseverbindung zwischen dem Maskenrahmen (400) und dem Band bilden, wobei sich der weibliche Abschnitt (470) im Allgemeinen zur Atemmaskenseite öffnet und bezüglich der Ausrichtung des Maskenrahmens (400) leicht abwärts und rückwärts abgewinkelt ist, so dass der weibliche Abschnitt (470) den männlichen Abschnitt (600, 700) annähernd parallel zu dem Teil der Wangenregion des Patienten hält, der von dem Abschnitt (600, 700) überlagert wird.

2. Atemmasken-Kopfband-Kombination nach Anspruch 1, wobei der Maskenrahmen (400) in Vorderansicht im Allgemeinen dreieckig ist, mit einer Grundfläche (410), einem Paar geneigter Seitenwände (420) die sich in Richtung eines Scheitelpunkts erstrecken und eine Stirnwand (430).

3. Atemmasken-Kopfband-Kombination nach Anspruch 2, wobei der Maskenrahmen (400) einen Rand (440) aufweist, der sich an der hinteren Kante der Grundfläche (410) und der Seitenwände (420) an die Gesichtskonturen des Patienten angleicht und für die Anbringung eines weichen Maskenkissens geeignet ist, um den Rahmen vom Gesicht des Patienten zu beabstanden.

4. Atemmasken-Kopfband-Kombination nach Anspruch 2 oder Anspruch 3, wobei der weibliche Abschnitt (470) integral in einem der unteren Eckbereiche des Maskenrahmens (400) geformt ist.

5. Atemmasken-Kopfband-Kombination nach einem der Ansprüche 1-4, wobei der männliche Abschnitt (600, 700) ein elastisch vorgespanntes Kragteil (660) aufweist, das von einem vorderen Endabschnitt (650) des männlichen Abschnitts (600, 700) abhängt und eine Einrichtung für den verschließenden Eingriff mit dem weiblichen Abschnitt (470) aufweist, wobei das Kragteil (660) ein vorderes Ende, ein hinteres Ende und eine Verriegelungseinrichtung aufweist, die zwischen dem vorderen Ende und dem hinteren Ende angebracht ist, um mit dem weiblichen Abschnitt (470) in Eingriff zu gelangen, und eine benachbart zum hinteren Ende liegende Löseeinrichtung, wobei die Löseeinrichtung einen erhöhten Abschnitt benachbart zum hinteren Ende des Kragteils (660) aufweist, wobei ein Raum unmittelbar hinter dem hinteren Endes des Kragteils (660) vorgesehen ist, wobei die Verriegelungseinrichtung mindestens einen Ansatz (610) auf einer vorderen Oberfläche des Kragteils (660) aufweist, wobei der Ansatz (610) mit einer entsprechenden Aufnahme (490) des weiblichen Abschnitts (470) in Eingriff steht.

6. Atemmasken-Kopfband-Kombination nach Anspruch 5, wobei der mindestens eine Ansatz (610) ein abgerundetes Keilprofil aufweist.

7. Atemmasken-Kopfband-Kombination nach Anspruch 2 oder nach einem der Ansprüche 3 bis 6 sofern diese von Anspruch 2 abhängig sind, wobei der Maskenrahmen (400) an seinem Scheitelpunkt eine Erweiterung (450) zur Anbringung einer Stirnstütze aufweist.

8. Atemmasken-Kopfband-Kombination nach einem der Ansprüche 1 bis 7, wobei der weibliche und männliche Abschnitt (470, 600, 700) vor dem Gesicht des Patienten durch den starren Maskenrahmen beabstandet sind.

9. Atemmasken-Kopfband-Kombination nach einem der Ansprüche 1 bis 8, wobei der männliche Abschnitt (600, 700) eine erste und eine zweite Griffflächen aufweist zum Greifen des männlichen Abschnitts (600, 700) mittels Daumen und Finger des Patienten, und eine Löseeinrichtung, die so angebracht ist, dass der Patient sie mit einem anderen Finger bedienen kann.

10. Atemmasken-Kopfband-Kombination nach Anspruch 5 oder nach einem der Ansprüche 6 bis 9 sofern diese von Anspruch 5 abhängig sind, wobei die Löseeinrichtung durch den Finger betätigt wird um das elastisch vorgespannte Kragteil (660) des männlichen Abschnitts (600, 700) zu drücken.

## Revendications

1. Combinaison d'un masque respiratoire et d'un harnais, comprenant :
un masque respiratoire avec une monture de masque (400) rigide, et
un harnais pour fixer le masque sur un patient, ledit harnais comportant au moins une sangle de fixation, une partie de connecteur femelle rigide (470) étant fixée de manière inamovible sur la monture de masque (400), comprenant en outre une partie de connecteur mâle (600, 700) correspondante prévue pour un accouplement amovible avec la partie de connecteur femelle (470), ladite partie de connecteur mâle (600, 700) étant raccordée à la sangle de fixation, la partie de connecteur femelle et la partie de connecteur mâle (470, 600, 700) formant une connexion déclenchable par pression entre la monture de masque (400) et la sangle, la partie de connecteur femelle (470) s'ouvrant sensiblement sur le côté du masque respiratoire mais étant légèrement inclinée vers le bas et vers l'arrière par rapport au plan de la monture de masque (400), de telle manière que la partie de connecteur femelle (470) maintient la partie de connecteur mâle (600, 700) sensiblement parallèle à la partie de la joue du patient recouverte par cette partie du partie du connecteur (600, 700).

2. Combinaison d'un masque respiratoire et d'un harnais selon la revendication 1, où la monture de masque (400) est sensiblement triangulaire en vue de face, avec une base (410), une paire de parois latérales (420) inclinées s'étendant vers un sommet et une paroi frontale (430).

3. Combinaison d'un masque respiratoire et d'un harnais selon la revendication 2, où la monture de masque (400) comporte un rebord (440) suivant les contours du visage du patient sur le bord arrière de la base (410) et les parois latérales (420), prévu pour la fixation d'un coussin de masque souple destiné à espacer la monture du visage du patient.

4. Combinaison d'un masque respiratoire et d'un harnais selon la revendication 2 ou la revendication 3, où la partie de connecteur femelle (470) est moulée d'un seul tenant avec une des zones de coin inférieur de la monture de masque (400).

5. Combinaison d'un masque respiratoire et d'un harnais selon l'une des revendications 1 à 4, où la partie de connecteur mâle (600, 700) présente un élément en porte-à-faux (660) contraint de manière élastique, dépendant d'une partie d'extrémité avant (650) de la partie de connecteur mâle (600, 700) et pourvu d'un moyen permettant un enclenchement de verrouillage avec la partie de connecteur femelle (470), où ledit élément en porte-à-faux (660) a une extrémité avant, une extrémité arrière, un moyen de verrouillage entre l'extrémité avant et l'extrémité arrière pour l'enclenchement avec la partie de connecteur femelle (470), et un moyen de déblocage adjacent à l'extrémité arrière, où ledit moyen de déblocage présente une partie en saillie adjacente à l'extrémité arrière de l'élément en porte-à-faux (660), où un espace est ménagé directement derrière l'extrémité arrière de l'élément en porte-à-faux (660), où le moyen de verrouillage comporte au moins un ergot (610) sur une surface avant de l'élément en porte-à-faux (660), ledit ergot (610) s'engageant dans une cavité (490) correspondante de la partie de connecteur femelle (470).

6. Combinaison d'un masque respiratoire et d'un harnais selon la revendication 5, où l'ergot ou les ergots (610) ont un profil en coin arrondi.

7. Combinaison d'un masque respiratoire et d'un harnais selon la revendication 2 ou selon l'une des revendications 3 à 6 si celle-ci est dépendante de la revendication 2, où la monture de masque (440) comprend une extension (450) à son sommet, prévue pour fixation sur un support frontal.

8. Combinaison d'un masque respiratoire et d'un harnais selon l'une des revendications 1 à 7, où la partie de connecteur femelle et la partie de connecteur mâle (470, 600, 700) sont séparées à l'avant du visage du patient par la monture de masque (400) rigide.

9. Combinaison d'un masque respiratoire et d'un harnais selon l'une des revendications 1 à 8, où la partie de connecteur mâle (600, 700) a une première et une deuxième surfaces de prise positionnées pour manipulation de la partie de connecteur mâle (600, 700) par un pouce et un doigt de la main du patient, et où le moyen de déblocage est positionné pour manipulation par un autre doigt de la main du patient.

10. Combinaison d'un masque respiratoire et d'un harnais selon la revendication 5 ou selon l'une des revendications 6 à 9 si celle-ci est dépendante de la revendication 5, où le moyen de déblocage subit une pression de doigt pour déclencher l'élément en porte-à-faux (660) contraint de manière élastique de la partie de connecteur mâle (600, 700).
